**Europäisches Patentamt**

**European Patent Office**

**Office européen des brevets**

(19)

(11) Veröffentlichungsnummer: **0 185 229**
**B1**

(12) **EUROPÄISCHE PATENTSCHRIFT**

(45) Veröffentlichungstag der Patentschrift:
15.02.89

(51) Int. Cl.⁴: **A 61 G 15/00,** A 61 B 6/00

(21) Anmeldenummer: **85115015.1**

(22) Anmeldetag: **27.11.85**

(54) **Parallelogrammtragarm mit Kräfteausgleichsvorrichtung.**

(30) Priorität: **10.12.84 DE 3445016**

(43) Veröffentlichungstag der Anmeldung:
**25.06.86 Patentblatt 86/26**

(45) Bekanntmachung des Hinweises auf die Patenterteilung:
**15.02.89 Patentblatt 89/7**

(84) Benannte Vertragsstaaten:
**AT DE FR GB IT SE**

(56) Entgegenhaltungen:
**FR-A-1 455 288**
**FR-A-1 577 416**
**FR-A-2 501 496**
**GB-A-724 471**
**GB-A-850 239**
**US-A-4 166 602**

(73) Patentinhaber: **Siemens Aktiengesellschaft Berlin und München, Wittelsbacherplatz 2, D-8000 München 2 (DE)**

(72) Erfinder: **Kiesel, Helmut, Schillerstrasse 3, D-6140 Bensheim 3 (DE)**
Erfinder: **Stöckl, Klaus, Franz- Schubert- Strasse 10, D-6140 Bensheim 3 (DE)**

## Beschreibung

Die Erfindung geht aus von einem Parallelogrammtragarm mit Kräfteausgleichsvorrichtung, wie er aus der US-A-4 166 602 zur Halterung eines Röntgengerätes vorgesehen ist. Bei diesem bekannten Parallelogrammtragarm ist der eine Gestängearm als Hohlarm ausgebildet, in dessen Innerem eine den zweiten Gestängearm bildende Stabilisierungsstange sowie eine Gasfeder angeordnet sind. Die beiden Enden der Stabilisierungsstange und des Hohlarmes sind mittels Achslagerungen mit Tragarm-Anschlußteilen gelenkig verbunden; diese Achslagerungen bilden das Gelenkviereck des Parallelogrammtragarmes.

Die Gasfeder ist unterhalb der Stabilisierungsstange im Hohlarm angeordnet, wobei Zylinder- und Kolbenstange der Gasfeder jeweils mit einem geeigneten Endstück versehen sind, von denen das eine mittels Gelenkachse an einem am Hohlarm angeformten Joch und das andere mittels einer Verstellschraube an einem am Tragarm-Anschlußteil befestigten Gehäuse angelenkt ist. Die von der Gasfeder erzeugte Kraft wirkt somit zwischen zwei diagonal liegenden Gelenkpunkten des Parallelogrammvierecks. Mit der Verstellschraube kann der Angriffspunkt der Kolbenstange verändert und so das Kräftemoment geändert und somit unterschiedlichen Gewichtslasten angepaßt werden. Die Endstücke sind mittels Schraubverbindung mit der Kolbenstange bzw. mit dem Zylinder verbunden, d. h. die Enden von Zylinder und Kolbenstange müssen auf die Endstücke und damit auf die Konstruktion des Parallelogrammtragarmes abgestimmt sein.

Wenngleich sich zwar durch die Verwendung einer Gasfeder der mechanische Aufwand gegenüber herkömmlichen, mit Druckfedern, Ketten und Kurvenelementen arbeitenden Tragarmkonstruktionen deutlich verringern läßt, so ist die bekannte Tragarmkonstruktion doch noch vergleichsweise aufwendig, insbesondere im Hinblick auf Montage und Demontage der Gasfeder sowie Einsatz des Tragarmes zum Tragen anderer Objekte mit anderen, außerhalb des mittels der Verstellschraube einstellbaren Bereichs liegender Gewichtslasten, denn mit der obengenannten bekannten Tragarmkonstruktion kann eine Kompensation der Gewichtslast nur in relativ engen, im wesentlichen im Rahmen der für die Tragarmkonstruktion vorgesehenen Gewichtslast erzielt werden. Ein Auswechseln der Gasfeder ist bei dieser bekannten Konstruktion nicht so ohne weiteres möglich.

Der im Anspruch 1 angegebenen Erfindung liegt die Aufgabe zugrunde, demgegenüber eine verbesserte, insbesondere noch einfachere und kostensparendere Tragarmkonstruktion anzugeben, die es gestattet, ein- und denselben Tragarm ohne Veränderung der Konstruktion und mit relativ geringem Montageaufwand zum Tragen von unterschiedlichen Objekten mit unterschiedlich großen Gewichtslasten einzusetzen, und die es ferner ermöglicht, auch in ihrer Länge unterschiedliche Tragarme unter Verwendung möglichst vieler gleicher Teile für unterschiedliche Anwendungs- und Belastungsfälle vorsehen zu können.

Dadurch, daß zwischen Gasfeder und den Endstücken der Parallelogrammarme keine feste, auf die spezielle Tragarmkonstruktion abgestimmte Verbindung besteht, kann die Gasfeder sehr leicht ausgewechselt und gegebenenfalls durch eine andere mit anderem Fülldruck ersetzt werden, wodurch für unterschiedlich hohe Belastungen Tragarme gleicher Länge eingesetzt werden können. Je nach Anwendungsfall braucht, ohne daß die Tragarmkonstruktion geändert werden muß, lediglich die in ihren Abmessungen gleichbleibende Gasfeder ausgewechselt zu werden. Durch die nachfolgend näher beschriebene Tragarmkonstruktion ist dies sehr leicht möglich. Gegebenenfalls kann ein Auswechseln sogar vom Kunden selbst vorgenommen werden, was insbesondere von Bedeutung ist für den Einsatz des Tragarmes in Ländern mit nicht so dichtem Servicenetz.

Die Gasfeder ist vorteilhafterweise in einem am Umfang geschlossenen Führungskanal eines Profils angeordnet und deshalb von außen nicht sichtbar.

Weitere vorteilhafte Ausgestaltungen und Weiterbildungen der Erfindung sind aus den Unteransprüchen zu entnehmen. Mehrere Ausführungsbeispiele der Erfindung werden nachfolgend anhand der Zeichnung näher erläutert:

Es zeigen:

Figur 1 einen Anwendungsfall für die erfindungsgemäße Tragarmkonstruktion in einer Prinzipdarstellung,

Figur 2 den Parallelogrammtragarm nach Figur 1 teilweise im Schnitt,

Figur 3 einen Querschnitt vom Profil für das untere Tragrohr mit aufgesetzter Abdeckung,

Figuren 4 bis 6 das eine in Figur 2 gezeigte Endstück 14 in Seitenansicht, Draufsicht und Frontansicht

Figur 7 eine Einzelheit (Bremseinrichtung) aus der Tragarmkonstruktion im Längsschnitt,

Figur 8 eine alternative Ausbildung einer Bremseinrichtung in schematischer Darstellung,

Figur 9 eine weitere Variante einer Bremseinrichtung im Schnitt,

Figuren 10 und 11 Teile eines Winkelstückes,

Figuren 12 und 13 Querschnitte entlang der Linien XII/XII bzw. XIII/XIII in Figuren 10 und 11.

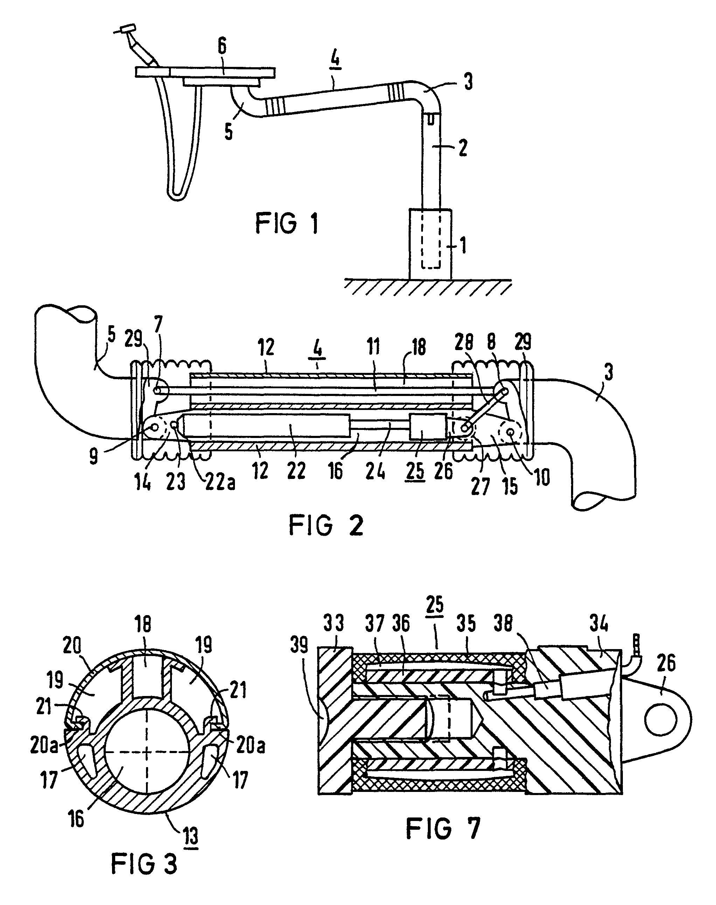

Die Figur 1 zeigt in einer Prinzipdarstellung einen im zahnärzlichen Bereich liegenden Anwendungsfall für die erfindungsgemäße Parallelogrammtragarmkonstruktion. An einem bodenseitig befestigten, ständerartigen Basisteil 1 ist ein vertikal verlaufendes Tragrohr 2 befestigt, an dessen oberen Ende ein Winkelstück 3 aufgesetzt ist, an dem das eine Ende eines Parallelogrammtragarmes 4 angelenkt ist, an dessen anderem Ende ein weiteres Winkelstück 5 angelenkt ist, an dem ein zahnärztlicher Instrumententisch 6 befestigt ist. Der Instrumententisch 6 läßt sich mittels des Parallelogrammtragarmes 4 in bekannter Weise in einem bestimmten Bereich in der Höhe verstellen. Anstelle des Instrumententisches kann auch ein anderes Gerät, z. B. ein Röntgengerät oder eine Leuchte, gehaltert sein. Letztere werden in der Regel nicht an einem bodenseitigen Ständer, sondern an einer Deckenaufhängung gehalten.

Anhand der Figur 2 wird der Aufbau des Parallelogrammtragarmes 4 näher erläutert. Das Parallelogrammgestänge wird gebildet durch eine horizontal geführte Stange 11, welche die beiden oberen Gelenkachsen 7, 8 der vier Gelenkachsen 7 bis 10 des Parallelogramms miteinander verbindet, und einen rohrförmigen Gestängearm 12, der die unteren Gelenkachsen 9, 10 miteinander verbindet. Der Gestängearm 12 besteht aus einem in Figur 3 im Querschnitt dargestellten rohrförmigen Profil 13 mit stirnseitig daran befestigten, die Lagerbuchsen für die Gelenkachsen 9 und 10 aufnehmenden Endstücken 14 und 15.

Das Profil 13 bildet mehrere längs verlaufende, zum Teil geschlossene, zum Teil zur Peripherie hin offene Führungskanäle 16 bis 19. Im einzelnen sind dies ein zentraler, mittig gelegener, im Querschnitt kreisförmiger Führungskanal 16, zwei symmetrisch dazu angeordnete, im Querschnitt etwa dreieckförmige Führungskanäle 17, ein oberhalb des Führungskanals 16 gelegener, nach oben offener Führungskanal 18 und zwei beidseitig daran angeordnete, ebenfalls zur Peripherie hin offene Führungskanäle 19. Die Schlitze der nach außen hin offenen Führungskanäle 18 und 19 werden durch eine längs des Tragarmes verlaufende Abdeckkappe 20, die mit ihrem Rand 20a in eine im Profil 13 vorgesehene Nut 21 eingreift, abgedeckt. Im oberen Führungskanal 18 ist die Parallelogrammstange 11 geführt, die beiden äußeren Führungskanäle 19 dienen zur Führung von diversen elektrischen und/oder pneumatischen und/oder hydraulischen Versorgungsleitungen, die von einem Anschlußkasten im Basisteil 1 zu den Verbrauchern, im vorliegenden Fall also zu den am Instrumententisch 6 gehalterten Handstücken, zu führen sind. Bei abgenommener Abdeckkappe 20 können die Leitungen innerhalb des Tragarmes besonders leicht verlegt bzw. bei Bedarf ausgewechselt werden. Zur Durchführung der Leitungen innerhalb der Winkelstücke 3 und 5 sind diese, wie später noch näher erläutert, ebenfalls mit abnehmbaren Abdeckkappen versehen, die nach Abnehmen einen guten Zugang zu den Leitungen gewährleisten. Wie aus Figur 3 hervorgeht, bildet die Abdeckkappe 20 mit dem Profil 13 eine kreisförmige Außenkontur, wobei die Abdeckkappe 20 etwa den halben Umfang des Profils bedeckt. Als Profil ist vorteilhafterweise ein Strangpreßprofil verwendet, die Abdeckkappe kann zweckmäßigerweise aus Kunststoff bestehen.

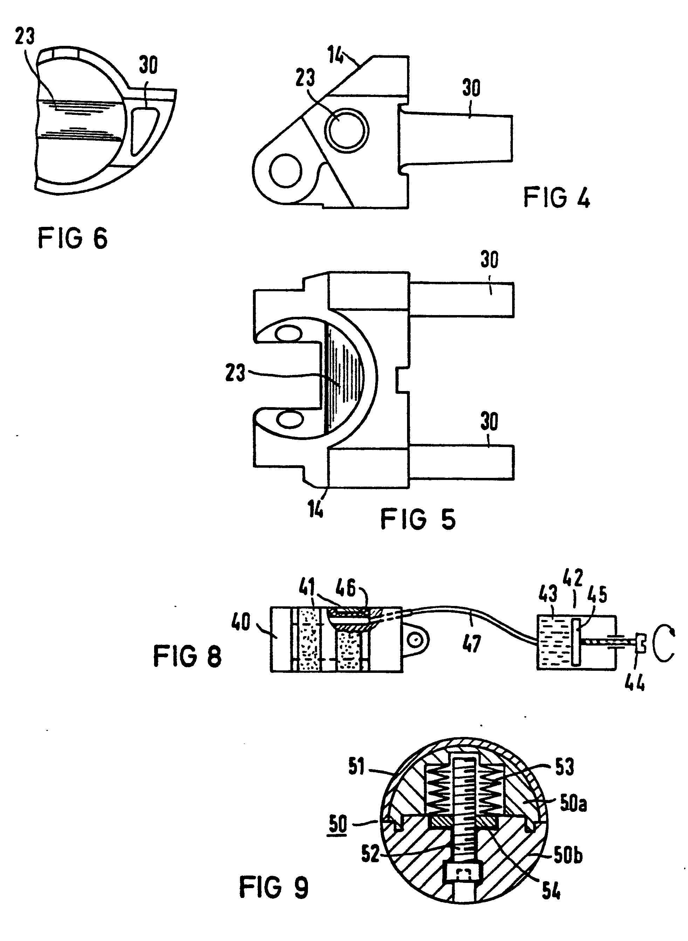

In den peripher geschlossenen Führungskanal 16 ist eine Gasfeder 22 eingelegt (Figur 2). Das eine Ende 22a stützt sich an einem im Endstück 14 angeordneten Querbolzen 23 ab, das freie Ende der Kolbenstange 24 liegt an der einen Stirnseite einer später noch näher erläuterten Bremseinrichtung 25 an, deren andere Stirnseite einen Ansatz 26 bildet, an dem einerseits eine Führungsrolle 27 gelagert und andererseits eine mit der Gelenkachse 8 verbundene Gelenkstange 28 angelenkt sind. Eine Demontage der Gasfeder, beispielsweise um diese gegen eine mit stärkerer Druckkraft (für eine höhere Gewichtslast) auszutauschen, ist sehr leicht durchführbar. Nach Lösen der Achse 9 kann der Tragarm 4 um die Lagerachse 7 geschwenkt werden. Entfernt man dann den Querbolzen 23, kann die Gasfeder durch das nach vorne offene Endstück 14 (Figuren 5/6) herausgezogen werden. Die Gelenkachsen zwischen den Parallelogrammgestängearmen und den beiden Winkelstücken 3 und 5 werden durch einen Faltenbalg 29 abgedeckt.

Die beiden Endstücke 14, 15 enthalten, wie in Figuren 4 bis 6 anhand des Endstückes 14 gezeigt ist, beidseitig im Querschnitt dem Querschnitt der Führungskanäle 17 entsprechende Steckzapfen 30; sie können nach Einführen in die Kanäle 17 mit dem Rohrprofil 13 in geeigneter Weise, vorteilhafterweise durch Kleben, mit diesen fest verbunden werden.

Alternativ ist es auch denkbar, das Endstück 14 nicht fest mit dem Profil 13 zu verbinden; vielmehr nur die Zapfen 30 in die Führungskanäle 17 einzustecken. Damit ergibt sich eine Variante zu der bereits geschilderten Auswechselbarkeit der Gasfeder. Anstelle einer Entnahme des Bolzens 23 kann alternativ das gesamte Endstück aus dem Profil entfernt werden.

Anhand der Figur 7 wird der Aufbau der Bremseinrichtung 25 näher erläutert. Die Bremseinrichtung 25 besteht im wesentlichen aus zwei miteinander verschraubten Kunststoffteilen 33, 34 und einem gummielastischen Bremselement 35, welches in einer durch eine Abstufung im Durchmesser der Kunststoffteile 33, 34 gebildeten ringförmigen Ausnehmung eingelegt ist. Das Bremselement 35 bildet zusammen mit einem ringförmigen Kunststoffteil 36 eine Druckkammer 37, die über einen Leitungskanal 38 mit einer Druckluftquelle in Verbindung gebracht werden kann. Das Kunststoffteil 33 weist stirnseitig eine Einkerbung 39 auf, in der sich das Ende der Kolbenstange 24 abstützen kann.

Die Bremseinrichtung dient dazu, den Tragarm bei zusätzlicher Gewichtsbelastung, z. B. bei vorübergehend auf dem Instrumententisch 6 abgelegten Gerätschaften, wie Filmbetrachter, Zahnsteinentfernungsgerät etc., in einer gewünschten Stellung arretieren zu können. Hierzu kann zweckmäßigerweise in Nähe des Instrumententisches 6 ein Ventil zur Freigabe von Druckluft aus einer Druckluftquelle an die Kammer 37 vorgesehen sein. Bei Druckluftbeaufschlagung legt sich die bei Nichtbeaufschlagung gegenüber den Mantelflächen der Kunststoffteile 33, 34 etwas zurückgesetzt angeordnete Mantelfläche des Bremselements 35 an den Wandungen des rohrförmigen Kanals 16 an. In dieser Stellung ist das Teil 35 blockiert und keine gewollte Verschiebung bzw. Verstellung des Tragarmes mehr möglich.

Für Fälle, in denen eine gewollte Verstellung angebracht ist, können anstelle des Bremselements 35 - oder auch zusätzlich zu diesem - ein oder mehrere Reibelemente vorgesehen sein, deren Reibkraft auf einen definierten Wert, gegebenenfalls variierbar, einstellbar ist. Die Einstellung kann dabei mechanisch, pneumatisch oder hydraulisch erfolgen. Drei Varianten sind in den Figuren 8 und 9 schematisch aufgezeigt.

Bei der Variante nach Figur 8 ist eine ähnlich der in Figur 7 aufgebaute Bremseinrichtung 40 vorgesehen; zusätzlich zu dem gummielastischen Bremselement 35 in Figur 7 sind bei dieser Ausführung mehrere, am Umfang angeordnete Reibelemente 41 vorgesehen, deren Reibkraft gegenüber der Rohrwandung 16 hydraulisch eingestellt werden kann. Hierzu ist eine extern des Tragarmes, vorzugsweise in Nähe des Instrumententisches 6, angeordnete, mit 42 bezeichnete hydraulische Stelleinrichtung vorgesehen, mittels der die Reibkraft der Bremselemente 41 eingestellt werden kann. Die Stelleinrichtung 42 besteht aus einem mit einer geeigneten Flüssigkeit gefüllten Zylinder 43 und einem mittels Stellschraube 44 von Hand verstellbaren Kolben 45 sowie einer in eine der Kammer 37 in Figur 7 entsprechende Kammer 46 der Bremseinrichtung 40 führenden Leitung 47. Mittels der Stellschraube 44 kann über den Kolben 45 die Hydraulikflüssigkeit auf den für die Einstellung der Reibkraft erforderlichen Druck eingestellt werden.

Anstelle der hydraulischen Einstellung kann auch eine mechanische Einstellung der Reibkraft vorgesehen werden. Eine mögliche Ausführung ist in Figur 9 dargestellt. Die Bremseinrichtung 50 besteht hier aus zwei Halbschalen 50a, 50b, von denen die erstere aus Reibbelag bestehen, mit einem Reibbelag versehen oder ein oder mehrere Reibelemente enthalten kann. Die im Ausführungsbeispiel mit einem Reibbelag 51 versehene Hälfte 50a wird mittels in der anderen Halbschale 50b angeordneter Stellglieder, z. B. in Form einer in einer Gewindemutter 54 geführten Innensechskantschraube 52, unter Zwischenlage von mehreren federnden Elementen, z. B. Tellerfedern 53, im montierten Zustand gegen die Wandung des Kanals 16 gedrückt. Eine solche mechanische Einstellung der Reibkraft ist dann angebracht, wenn die am Tragarm angreifende Gewichtslast weitgehend fest ist, jedoch eine Verstellung des Tragarmes bei der vorgesehenen Belastung mit individueller Schwergängigkeit eingestellt werden soll.

Anstelle der extern blockierbaren Gasfedern können auch Gasfedern mit integriert angeordneten steuerbaren Ventilen vorgesehen werden, die mit Betätigen der Kolbenstange ausgelöst werden. Bei Verwendung solcher Gasfedern ist es vorteilhaft, eine pneumatische Steuereinrichtung vorzusehen, um den Auslösestößel der Gasfeder für die Freigabe bzw. Sperrung des Gasaustausches innerhalb der Kammern in der Gasfeder steuern zu können.

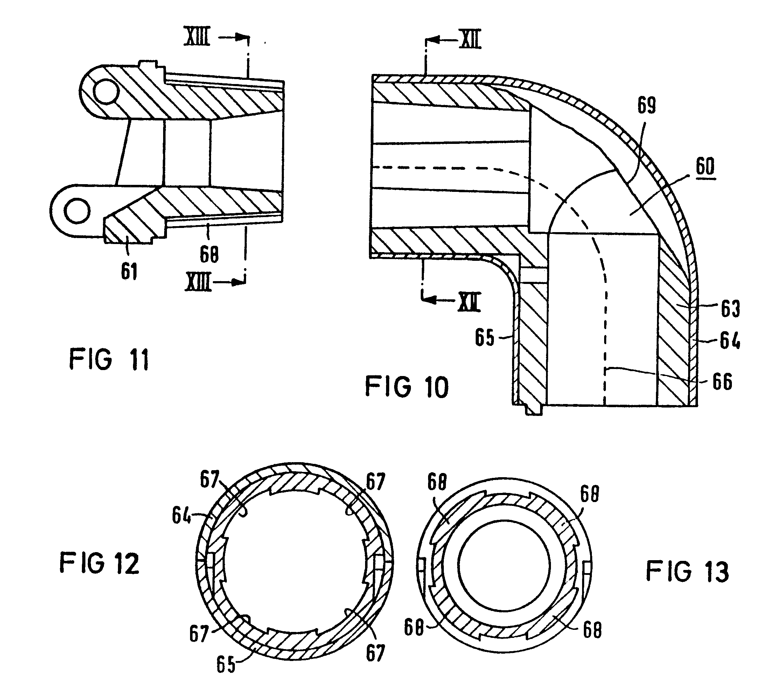

Anhand der Figuren 10 bis 13 wird der Aufbau der Winkelstücke 3 bzw. 5 näher erläutert. Beide Winkelstücke sind unter Verwendung gleicher Bauteile aufgebaut; sie bestehen aus einem in Figur 10 im Längsschnitt dargestellten Rohrbogen 60 und einem am einen Ende des Rohrbogens aufgesetzten, in Figur 11 im Schnitt dargestellten Gelenkkopf 61. Der Rohrbogen 60 seinerseits besteht aus einem Gußkörper 63 und zwei abnehmbar daran gehalterten Halbschalen 64, 65, die an der gestrichelt eingezeichneten Trennlinie 66 in geeigneter Weise, z. B. durch federnde Rastelemente, miteinander verbunden sind. Dieser Aufbau hat mehrere Vorzüge; dadurch, daß der äußere, sichtbare Teil des Bogens durch die beiden Kunststoffhalbschalen gebildet wird, braucht die Oberfläche des Gußkörpers selbst nicht weiter nachbearbeitet zu werden. Insbesondere entfallen relativ aufwendige Schleif-, Spachtel- und Lackierarbeiten. Unterschiedliche Farbwünsche können bei der Erstellung der Kuststoffhalbschalen berücksichtigt werden.

Der Gußkörper 63 weist am dem Tragarm zugewandten Ende den in Figur 12 gezeigten Querschnitt auf. Hieraus ist ersichtlich, daß der Gußkörper vier am Umfang gleichmäßig verteilt angeordnete, konisch zum Bogen zulaufende Nuten 67 aufweist, in die entsprechende, längs verlaufende Rippen 68 eingreifen, die der in Figur 11 im Längsschnitt dargestellte Gelenkkopf 61, der die nicht näher bezeichneten Lagerbuchsen zur Aufnahme der Gelenkachsen 8 und 10 (Figur 2) enthält, aufweist.

Auf den Gelenkkopf 61 kann der Rohrbogen 60 nun entweder, wie in Figur 10 gezeigt, mit nach unten weisendem Rohrende oder um 180° versetzt, d. h. mit nach oben gerichtetem Rohrende, aufgesetzt werden. Nach dem Zusammenstecken der Teile können diese, sofern erwünscht, in geeigneter Weise, z. B. durch Verkleben, Verstiften, dauerhaft haltbar miteinander verbunden werden.

Der Gußkörper 63 enthält im Bereich des Bogens eine Ausnehmung 69, die im aufgesetzten Zustand durch die Halbschale 64 verdeckt wird. Nach Abnehmen der Halbschale 64 ist so ein Zugang von außen zum Bogeninneren gegeben, wodurch sich z. B. innerhalb der Tragrohranordnung 1 bis 5 verlegte Leitungen sehr leicht auswechseln lassen.

**Patentansprüche**

1. Parallelogrammtragarm mit Kräfteausgleichsvorrichtung unter Verwendung einer in einem rohrförmigen Führungskanal (16) des einen Gestängearmes (12) leicht auswechselbar angeordneten Gasfeder (22), deren eines Ende (Zylinder oder Kolbenstange (24)) sich an einem mit der einen Gelenkachse (9) verbundenen Endstück (14) und deren anderes Ende (Kolbenstange (24) oder Zylinder) sich an mit der anderen, diagonal gegenüberliegend angeordneten Gelenkachse (8) verbundenen Teilen (25, 26, 28) abstützen, wobei zur leichten Auswechselbarkeit der Gasfeder (22) das Endstück (14) am einen stirnseitigen Ende des Führungskanals (16) lösbar oder fest gehalten und mit der benachbarten Gelenkachse (9) lösbar verbunden ist.

2. Parallelogrammtragarm nach Anspruch 1, dadurch gekennzeichnet, daß zum Kräfteausgleich von unterschiedlichen Gewichtslasten in den Außenabmessungen gleiche, im Fülldruck jedoch veränderbare Gasfedern (22) verwendet sind.

3. Parallelogrammtragarm nach Anspruch 1, dadurch gekennzeichnet, daß die Kolbenstange (24) mittels einer Gelenkstange (28) mit der diagonal angeordneten Gelenkachse (8) verbunden ist, und Zylinder und/oder Kolbenstange (24) sich an einem für Demontagezwecke lösbar angeordneten Kraftübertragungsglied (23, 25) abstützen.

4. Parallelogrammtragarm nach Anspruch 3, dadurch gekennzeichnet, daß sich das freie Ende der Kolbenstange (24) an einer Bremseinrichtung (25) abstützt, die unter Zwischenlage einer Gelenkverbindung (26, 28) mit der einen Gelenkachse (8) verbunden ist, daß sich das der Kolbenstange (24) abgewandte Ende (22a) der Gasfeder (22) an einem Kraftübertragungsglied (23) des mit der diagonal angeordneten anderen Gelenkachse (9) lösbar verbundenen Endstückes (14) abstützt.

5. Parallelogrammtragarm nach Anspruch 4, dadurch gekennzeichnet, daß der eine Gestängearm (12) aus einem, mehrere Längskanäle (16 bis 19) aufweisenden rohrförmigen Profil (13) mit beidseitig am stirnseitigen Ende aufgesetzten, die einen Gelenkachsen (9, 10) aufnehmenden Endstücken (14, 15) gebildet ist und der andere Gestängearm (11) in einem (18) der Längskanäle (16 bis 19) geführt ist.

6. Parallelogrammtragarm nach Anspruch 5, dadurch gekennzeichnet, daß das Profil (13) so gestaltet ist, daß einige der Längskanäle (18, 19) an ihrer Peripherie offen sind und so längs verlaufende Schlitze bilden, und daß eine auf das Profil formschlüssig und/oder kraftschlüssig aufsetzbare Abdeckung (20) vorhanden ist, welche im aufgesetzten Zustand die Schlitze abdeckt.

7. Parallelogrammtragarm nach Anspruch 6, dadurch gekennzeichnet, daß zur Befestigung der Abdeckung (20) das Profil (13) längsseitig Nuten (21) aufweist, in die die Ränder (20a) der Abdeckung (20) eingeklemmt sind.

8. Parallelogrammtragarm nach Anspruch 6, dadurch gekennzeichnet, daß Profil (13) und Abdeckung (20) im montierten Zustand eine kreisförmige Außenkontur aufweisen und die an der Peripherie offenen Kanäle (18, 19) am halben Umfang des Profils (13) angeordnet sind.

9. Parallelogrammtragarm nach Anspruch 5, dadurch gekennzeichnet, daß das Profil (13) einen im Querschnitt kreisförmigen ersten Führungskanal (16), in den die Gasfeder (22) einlegbar ist, beidseitig davon symmetrisch angeordnete zweite Führungskanäle (17), in die Steckzapfen (30) zur Halterung der Endstücke (14, 15) eingreifen, und einen oberhalb des ersten Führungskanals (16) angeordneten dritten Führungskanal (18), in dem der andere Gestängearm (11) geführt ist, enthält.

10. Parallelogrammtragarm nach Anspruch 4, dadurch gekennzeichnet, daß die Bremseinrichtung ein oder mehrere Reibelemente (35, 41, 51) enthält, deren gegen die Wandung des Führungskanals (16) gerichteter Anpreßdruck durch Stellglieder (52, 53, 54) einstellbar ist.

11. Parallelogrammtragarm nach Anspruch 10, dadurch gekennzeichnet, daß die Bremseinrichtung einen hydraulisch oder pneumatisch beaufschlagbaren Bremskörper (25, 41) mit einem gummielastischen, mit dem Druckmittel beaufschlagbaren Formteil (35) enthält, welches in einem im Durchmesser abgestuften zylindrischen Körper (33, 34) eingelegt ist.

12. Parallelogrammtragarm nach Anspruch 10, dadurch gekennzeichnet, daß die Bremseinrichtung aus zwei gegeneinander verstellbaren Halbschalen (50a, 50b) gebildet ist, von denen wenigstens eine aus Reibmaterial besteht oder mit einem Reibbelag bzw. mit Reibelementen (51) versehen ist.

13. Parallelogrammtragarm nach Anspruch 10, dadurch gekennzeichnet, daß der Bremskörper (41) hydraulisch verstellbar ist und die Reibkraft durch extern des Tragarmes angeordnete mechanische Stellmittel (44, 45) einstellbar ist.

14. Parallelogrammtragarm nach Anspruch 1, dadurch gekennzeichnet, daß die Gelenkachsen (7, 9; 8, 10) der Parallelogrammgestängearme (11, 12) an Winkelstücken (3, 5) angeordnet sind, die aus einem Rohrbogen (60) mit Innenprofil (67) und einem die Gelenkachsen aufnehmenden Gelenkkopf (61) mit symmetrischem, zum Innenprofil (67) passenden Außenprofil (68) bestehen.

15. Parallelogrammtragarm nach Anspruch 14, dadurch gekennzeichnet, daß die Profile (67, 68) vom Gelenkkopf (61) und Rohrbogen (60) eine Teilung aufweisen, die es ermöglicht, den Gelenkkopf (61) um 180° versetzt in den Rohrbogen (60) einzusetzen.

16. Parallelogrammtragarm nach Anspruch 15,

dadurch gekennzeichnet, daß der Rohrbogen (60) im Bereich des Bogens eine durch eine abnehmbare Abdeckung (64) zugängliche Ausnehmung (69) aufweist.

17. Parallelogrammtragarm nach Anspruch 16, dadurch gekennzeichnet, daß der Rohrbogen (60) aus einem Gußkörper (63) und zwei dessen Mantelfläche verkleidenden Halbschalen (64, 65) besteht, von denen zumindest diejenige (64), welche die Ausnehmung (69) bedeckt, leicht abnehmbar am Gußkörper (63) angeordnet ist.

**Claims**

1. A parallelogram supporting arm with a counterbalancing mechanism using a pneumatic spring (22), arranged in a tubular guide passage (16) of a linkage arm (12) so that it can be exchanged easily, whose one end (cylinder or piston rod (24)) bears against an end-piece (14) connected to one axis (9) of the linkage, and whose other end (piston rod (24) or cylinder) bears against parts (25, 26, 28) connected to the other, diagonally opposed axis (8) of the linkage, and for easy exchangeability of the pneumatic spring (22) the end-piece (14) is detachably mounted on or fixed to one end of the guide passage (16) and is connected detachably to the adjacent axis (9) of the linkage.

2. A parallelogram supporting arm according to claim 1, characterised in that in order to counterbalance different weight loads pneumatic springs (22) are used which have the same external dimensions but whose filling pressure can be varied.

3. A parallelogram supporting arm according to claim 1, characterised in that the piston rod (24) is connected to the diagonally located axis (8) of the linkage by means of a linkage rod (28), and the cylinder and/or piston rod (24) bear on a force-transmission member (23, 25) which is detachable for dismantling purposes.

4. A parallelogram supporting arm according to claim 3, characterised in that the free end of the piston rod (24) bears on a braking device (25) which is connected to the one axis (8) of the linkage through an intermediate linkage connection (26, 28), and in that the end (22a) of the pneumatic spring (22) facing away from the piston rod (24) bears on a force-transmission member (23) of the end-piece (14) connected detachably to the other diagonally located axis (9) of the linkage.

5. A parallelogram supporting arm according to claim 4, characterised in that the one linkage arm (12) is formed by a tubular section (13) having a plurality of longitudinal passages (16 to 19) and having endpieces (14, 15) mounted on both ends and accommodating one axis (9, 10) of the linkage, and the other linkage arm (11) passes through one (18) of the longitudinal passages (16 to 19).

6. A parallelogram supporting arm according to claim 5, characterised in that the section (13) is formed so that some of the longitudinal passages (18, 19) are open on their periphery and thus form longitudinal slits, and in that there is a cover (20) which can be attached to the section either in a form-fitting or friction-locked manner and covers the slits when it is mounted.

7. A parallelogram supporting arm according to claim 6, characterised in that in order to secure the cover (20) the section (13) has grooves (21) along the side in which the rims (20a) of the cover (20) are clamped.

8. A parallelogram supporting arm according to claim 6, characterised in that the section (13) and cover (20) have a circular external contour when assembled and the open passages (18, 19) on the periphery are arranged on half the circumference of the section (13).

9. A parallelogram supporting arm according to claim 5, characterised in that the section (13) comprises a first guide passage (16) of circular cross-section into which the pneumatic spring (22) can be inserted, second guide passages (17) arranged symmetrically on either side thereof into which dowel pins (30) engage to support the end-pieces (14, 15), and a third guide passage (18), arranged above the first guide passage (16), in which the other linkage arm (11) is guided.

10. A parallelogram supporting arm according to claim 4, characterised in that the braking device comprises one or more friction elements (35, 41, 51) whose pressure against the wall of the guide passage (16) can be adjusted by control members (52, 53, 54).

11. A parallelogram supporting arm according to claim 10, characterised in that the braking device comprises a braking body (25, 41), which can be acted on hydraulically or pneumatically, having a rubber elastic moulding (35) which can be acted on by the pressure means and which is inserted in a cylindrical body (33, 34) which is stepped in diameter.

12. A parallelogram supporting arm according to claim 10, characterised in that the braking device is made up of two mutually adjustable half-shells (50a, 50b) of which at least one consists of friction material or is provided with a friction coating or with friction elements (51).

13. A parallelogram supporting arm according to claim 10, characterised in that the braking body (41) is hydraulically adjustable and the friction force can be adjusted by mechanical adjusting means (44, 45) arranged outside of the supporting arm.

14. A parallelogram supporting arm according to claim 1, characterised in that the axes (7, 9; 8, 10) of the linkage of the parallelogram supporting arms (11, 12) are arranged on angle pieces (3, 5) which comprise a pipe bend (60) having an internal section (67) and a linkage head (61), accommodating the axes of the linkage, having a symmetrical external section (68) matching the internal section (67).

15. A parallelogram supporting arm according to claim 14, characterised in that the sections (67, 68) of the linkage head (61) and the pipe bend (60) are divided to enable the linkage head (61), turned through 180°, to be inserted in the pipe bend (60).

16. A parallelogram supporting arm according to claim 15, characterised in that the pipe bend (60) has a recess (69) in the region of the bend which is made accessible by a removable cover (64).

17. A parallelogram according to claim 16, characterised in that the pipe bend (60) comprises a casting (63) and two half-shells (64, 65) covering its surface, of which at least the one (64) covering the recess (69) is arranged so that it can be removed from the casting (63).

**Revendications**

1. Bras de support en forme de parallélogramme comportant un dispositif d'équilibrage des forces et comprenant un ressort pneumatique (22), qui est disposé, de manière à pouvoir être aisément remplacé, dans un canal tubulaire de guidage (16) d'un bras de tringlerie (12) et dont une extrémité (cylindre ou tige de piston (24)) prend appui sur une pièce d'extrémité (14) reliée à un axe d'articulation (9) et dont l'autre extrémité (tige de piston (24) ou cylindre) prend appui sur des parties (25, 26, 28) reliées à l'autre axe d'articulation (8) situé dans une position opposée en diagonale, et pour l'obtention d'une interchangeabilité aisée du ressort pneumatique (22), la pièce d'extrémité (14) est maintenue d'une manière fixe ou amovible sur une extrémité frontale du canal de guidage (16) et est reliée de façon amovible à l'axe d'articulation voisin (9).

2. Bras de support en forme de parallélogramme suivant la revendication 1, caractérisé par le fait que pour l'équilibrage des forces produites par des charges pondérales différentes, on utilise des ressorts pneumatiques (22) dont les dimensions extérieures sont les mêmes, mais dont les pressions de remplissage sont différentes.

3. Bras de support en forme de parallélogramme selon la revendication 1, caractérisé par le fait que la tige de piston (24) est reliée au moyen d'une barre articulée (28) à l'axe d'articulation (8) disposé en diagonale et que le cylindre et/ou la tige de piston (24) prend appui sur un organe de transmission de force (23, 25) monté de façon amovible pour le démontage.

4. Bras de support en forme de parallélogramme suivant la revendication 3, caractérisé par le fait que l'extrémité libre de la tige de piston (24) prend appui sur un dispositif de freinage (25) qui est relié, moyennant l'interposition d'une liaison articulée (26, 28), à un axe d'articulation (8), que l'extrémité (22a) du ressort pneumatique (22) tourné à l'opposé de la tige de piston (24), prend appui sur un organe de transmission de force (23) de la pièce d'extrémité (14) reliée de façon amovible à l'autre axe d'articulation (9) disposé en diagonal.

5. Bras de support en forme de parallélogramme suivant la revendication 4, caractérisé par le fait qu'un bras de tringlerie (12) est constitué par un profilé tubulaire (13) comportant plusieurs canaux longitudinaux (16 à 19) et possédant des pièces d'extrémité (14, 15) montées sur son extrémité frontale et recevant chacune un axe d'articulation (9, 10), tandis que l'autre bras de tringlerie (11) est guidé dans l'un (18) des canaux longitudinaux (16 à 19).

6. Bras de support en forme de parallélogramme suivant la revendication 5, caractérisé par le fait que le profilé (13) est agencé de sorte que quelques-uns des canaux longitudinaux (18, 19) sont ouverts sur leur pourtour et forment ainsi des fentes longitudinales, et qu'il est prévu un capot (20) pouvant être placé sur le profilé selon une liaison par formes complémentaires et/ou une liaison de force et, à l'état installé, recouvre les fentes.

7. Bras de support en forme de parallélogramme suivant la revendication 6, caractérisé par le fait que pour la fixation du capot (20), le profilé (13) comporte des rainures longitudinales (21), dans lesquelles les bords (20a) du capot (20) sont fixés par serrage.

8. Bras de support en forme de parallélogramme suivant la revendication 6, caractérisé par le fait que le profilé (13) et le capot (20) possèdent, à l'état monté, un contour extérieur circulaire et que les canaux (18, 19) ouverts au niveau de la périphérie, sont disposés sur la moitié du pourtour du profilé (13).

9. Bras de support en forme de parallélogramme suivant la revendication 5, caractérisé par le fait que le profilé (13) contient un premier canal de guidage (16), dont la section transversale possède une forme circulaire et dans lequel le ressort pneumatique (22) peut être inséré, les seconds canaux de guidage (17), qui sont disposés, des deux côtés, symétriquement par rapport au premier canal de guidage et dans lesquels des tétons enfichables (30) s'engagent pour maintenir les pièces d'extrémité (14, 15), et un troisième canal de guidage (18), qui est disposé au-dessus du premier canal de guidage (16) et dans lequel l'autre bras de tringlerie (11) est guidé.

10. Bras de support en forme de parallélogramme suivant la revendication 4, caractérisé par le fait que le dispositif de freinage contient un ou plusieurs éléments de friction (35, 41, 51), dont la pression d'application dirigée vers la paroi du canal de guidage (16) peut être réglée au moyen d'organes de réglage (52, 53, 54).

11. Bras de support en forme de parallélogramme suivant la revendication 10, caractérisé par le fait que le dispositif de freinage possède un corps de freinage (25, 41) chargé d'une manière hydraulique ou pneumatique et comportant une pièce de forme (35) présentant l'élasticité du caoutchouc, pouvant être chargé par le fluide sous pression et inséré dans un corps cylindrique (33, 34) possédant un diamètre étagé.

12. Bras de support en forme de parallélogramme suivant la revendication 10, caractérisé par le fait quel le dispositif de freinage est formé de deux demi-coques (50a, 50b) déplaçables l'une par rapport à l'autre, dont l'une au moins est réalisée avec un matériau de friction ou bien est pourvue d'une garniture de friction ou d'éléments de friction (51).

13. Bras de support en forme de parallélogramme suivant la revendication 10, caractérisé par le fait que le corps de freinage (41) est déplaçable par voie hydraulique et que la force de friction est réglable à l'aide de moyens mécaniques de réglage (44, 45) disposés à l'extérieur du bras de support.

14. Bras de support en forme de parallélogramme suivant la revendication 1, caractérisé par le fait que les axes d'articulation (7, 9; 8, 10) des bras de tringlerie en forme de parallélogramme (11, 12) sont disposés sur des pièces coudées (3, 5) qui sont constituées par un coude tubulaire (60) possédant un profil intérieur (67) et une tête articulée (61) logeant les axes d'articulation et possédant un profil extérieur symétrique (68) adapté au profil intérieur (67).

15. Bras de support en forme de parallélogramme suivant la revendication 14, caractérisé par le fait que les profils (67, 68) de la tête articulée (62) et du coude tubulaire (60) possèdent une subdivision permettant d'insérer la tête articulée (61) en la décalant de 180° dans le coude tubulaire (60).

16. Bras de support en forme de parallélogramme suivant la revendication 15, caractérisé par le fait que le coude tubulaire (60) possède, au voisinage du coude, un évidement (69) accessible à travers une enveloppe amovible (64).

17. Bras de support en forme de parallélogramme suivant la revendication 16, caractérisé par le fait que le coude tubulaire (60) est constitué par un corps en fonte (63) et par deux demi-coques (64, 65), qui recouvrent la surface enveloppe du corps en fonte et dont au moins celle (64) qui recouvre l'évidement (69), est montée sur le corps en fonte (63) de manière à être aisément amovible.

**FIG 1**

**FIG 2**

**FIG 3**

**FIG 7**

FIG 6

FIG 4

FIG 5

FIG 8

FIG 9

EP 0 185 229 B1

XIII →

FIG 11

61    68

XIII →

← XII

69

60

65

XII

63

64

66

FIG 10

FIG 12

67    67

64

67

65    67

67

68    68

68

68    68

FIG 13